(19)

Europäisches
Patentamt
European
Patent Office
Office européen
des brevets

(11) **EP 3 244 210 A1**

(12) **EUROPEAN PATENT APPLICATION**

published in accordance with Art. 153(4) EPC

(43) Date of publication:
**15.11.2017 Bulletin 2017/46**

(21) Application number: **16735189.9**

(22) Date of filing: **07.01.2016**

(51) Int Cl.:
*G01N 33/569* $^{(2006.01)}$   *G01N 33/58* $^{(2006.01)}$
*G01N 21/17* $^{(2006.01)}$   *G01N 21/62* $^{(2006.01)}$
*C12Q 1/37* $^{(2006.01)}$

(86) International application number:
**PCT/KR2016/000153**

(87) International publication number:
**WO 2016/111568 (14.07.2016 Gazette 2016/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **08.01.2015  KR 20150002793
19.01.2015  KR 20150008955
19.01.2015  KR 20150008956**

(71) Applicants:
• **Huvet Bio, Inc.
Seoul 06247 (KR)**
• **Korea Research Institute of Bioscience and
Biotechnology
Daejeon 34141 (KR)**

(72) Inventors:
• **HAAM, Seungjoo
Seoul 03722 (KR)**
• **KIM, Hyun-Ouk
Seoul 03722 (KR)**
• **SONG, Daesub
Daejeon 34069 (KR)**
• **KIM, Jihye
Seoul 03722 (KR)**
• **JANG, Eunji
Seoul 03722 (KR)**
• **CHOI, Jihye
Incheon 22165 (KR)**

(74) Representative: **Vossius & Partner
Patentanwälte Rechtsanwälte mbB
Siebertstrasse 3
81675 München (DE)**

(54) **KIT FOR DETECTING VIRUS**

(57)    The present invention relates to a kit for detecting a virus, a composition for detecting a virus and a method for detecting a virus. According to the present invention, viruses may be detected with high efficiency at low cost within a short period of time.

FIG. 1

EP 3 244 210 A1

**Description**

[Technical Field]

The present invention relates to a kit for detecting a virus, a method for detecting a virus and a composition for detecting a virus.

[Background Art]

Viruses are contagious pathogens smaller than bacteria. A virus consists of RNA or DNA as a genetic material and a protein surrounding the genetic material. Viruses may be classified into plant viruses, animal viruses and bacterial viruses (phages) according to the type of host. However, in most cases, viruses are divided into DNA virus subphyla and RNA virus subphyla according to the type of nucleic acid, and also subdivided into class, order and family.

Among such viruses, avian influenza is an acute viral infectious disease caused by an infection by avian influenza viruses in chickens, ducks or wild birds, and rarely developing infectious diseases in humans. Avian influenza viruses are classified into three types of high pathogenicity, low pathogenicity and non-pathogenicity according to pathogenicity, and over 640 cases of human infections by the highly pathogenic Avian influenza A (H5N1) have been reported from late 2003 to February in 2008. Avian influenza causes great economic damage due to high mortality and low egg production in birds, and, while not highly likely to cause infection, has high mortality in humans when infected. Since it is difficult to develop a fundamental avian influenza vaccine and has a very high spreading rate, it is necessary to minimize the spread of the disease and economic loss through early diagnoses.

As methods for diagnosing such a virus, immunological detection methods such as enzyme-linked immunosorbent assay (ELISA), enzyme immunoassay (EIA) and immunofluorescence assay (IFA) and an RNA detection method by RT-PCR are known, however, these methods are problematic in that it takes too much time to diagnose a virus and requires high cost for experiments or specificity and sensitivity are decreased due to nonspecific reactions.

Therefore, it is necessary to develop a method for efficiently detecting a virus in a short period of time at low cost.

[Disclosure]

[Technical Problem]

The present invention is directed to providing a kit for detecting a virus, which can efficiently detect a virus within a short period of time, a method for detecting a virus, and a composition for detecting a virus.

[Technical Solution]

The present invention provides a kit for detecting a virus, which comprises a biomolecule that specifically reacts with a surface protein of a virus; and a probe that reacts with the virus activated by the biomolecule.

The present invention also provides a composition for detecting a virus, which comprises a biomolecule that specifically reacts with a surface protein of a virus; and a probe that reacts with the virus activated by the biomolecule.

The present invention also provides a method for detecting a virus, which comprises contacting a sample obtained from a subject with a biomolecule that specifically reacts with a surface protein of a virus, and contacting the sample in contact with the biomolecule with a probe that reacts with the virus activated by the biomolecule.

[Advantageous Effects]

According to the present invention, a virus can be detected at low cost with high efficiency in a short period of time.

[Description of Drawings]

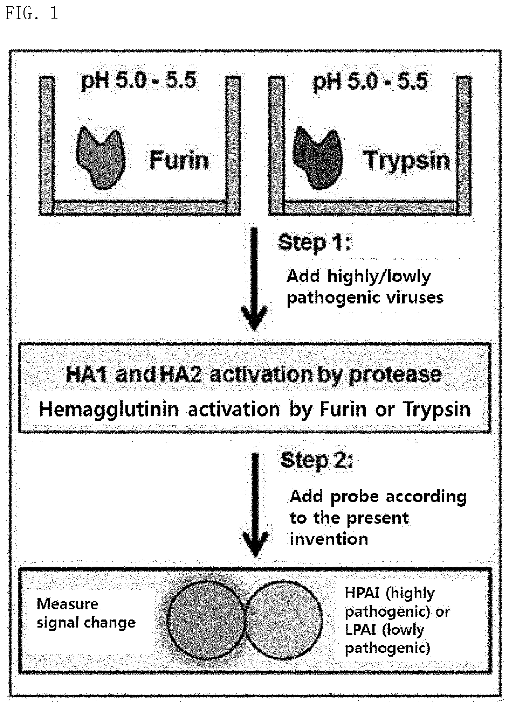

FIG. 1 is a schematic diagram of a method for detecting influenza virus according to an embodiment of the present invention.

FIG. 2 shows TEM images of amphiphilic particles (FluSome) according to an embodiment of the present invention.

FIG. 3 shows the mean diameter of amphiphilic particles according to an embodiment of the present invention.

FIG. 4 shows a result confirming the effect of fluorescence resonance energy transfer (FRET) for amphiphilic particles according to an embodiment of the present invention.

FIG. 5 shows a result of detecting influenza virus using amphiphilic particles according to an embodiment of the present invention.

FIG. 6 shows a result of detecting various types of influenza virus using amphiphilic particles according to an embodiment of the present invention.

FIG. 7A, 7B, 7C and 7D show a result of detecting various types of influenza virus using amphiphilic particles according to an embodiment of the present invention.

FIG. 8 shows a result of detecting influenza virus using organic/inorganic particles according to an embodiment of the present invention.

FIG. 9 shows a result of detecting influenza virus using organic/inorganic particles according to an embodiment of the present invention.

FIG. 10A, 10B and 10C show a result of detecting influenza virus through measurement of surface-enhanced Raman scattering signals.

FIG. 11 shows a result of detecting influenza virus through visual observation of color changes.

FIG. 12 shows a result of detecting influenza virus using liposomes according to an embodiment of the present invention.

[Modes of the Invention]

All types of viruses including influenza virus and Ebola virus consist of a surface protein hemagglutinin (HA) binding to a host cell for invasion of host cells, neuraminidase involved in escape of mature virions from the host cells, an M2 ion channel for controlling balance in hydrogen ion concentrations, and ribonucleoprotein (RNP) containing the genetic information of a virus.

Based on the common characteristics of the viruses described above, the inventors have conducted studies to develop a method for detecting a virus in an early stage. As a result, they found that viruses can be detected by a simple method using a biomolecule that specifically reacts with the surface protein of a virus to activate viruses and a probe that reacts with the virus activated thereby to detect the existence of the viruses, and the present invention was completed.

Therefore, the present invention provides a kit for detecting a virus, which comprises a biomolecule that specifically reacts with a surface protein of a virus and a probe that reacts with the virus activated by the biomolecule.

The term "virus" used herein refers to an obligatory intracellular parasite, which has DNA or RNA as a nucleic acid, starts proliferating from the nucleic acid, does not proliferate through binary fission, and does not have an enzyme system required for ATP production. The virus of the present invention encompasses a naked virus and an enveloped virus, and specifically, according to an embodiment of the present invention, the virus may be an enveloped virus.

Specifically, the enveloped virus encompasses DNA viruses such as herpesvirus, poxvirus and hepadnavirus, and RNA viruses such as flavivirus, togavirus, coronavirus, hepatitis D, orthomyxovirus, paramyxovirus, rhabdovirus, bunyavirus, filovirus and retrovirus.

The orthomyxovirus encompasses influenza virus A, influenza virus B, influenza virus C, isavirus, thogotovirus and quaranjavirus genera.

The coronavirus encompasses alpha coronavirus, beta coronavirus, gamma coronavirus and delta coronavirus genera.

The paramyxovirus encompasses paramyxovirus, rubella virus, morbillivirus and pneumovirus genera.

The virus includes a nucleic acid and a protein surrounding the nucleic acid. Such a viral protein is involved in protecting a viral genome, attaching and/or fusing virus particles to a cell, determines viral antigenicity, and includes a structural protein that maintains the shape of a virus and a non-structural protein that is associated with the synthesis of proteins and nucleic acids.

The term "surface protein of a virus" used herein refers to the structural protein. Specifically, the "surface protein" of the present invention has specific antigenicity of a virus, and encompasses all peripheral proteins that are present on an envelope involved in attaching and/or fusing a virus to a cell. In an embodiment, such a surface protein of the present invention may include a glycoprotein, and a fusion protein involved in fusion to a cell.

According to an embodiment of the present invention, the surface protein of the virus may be hemagglutinin (HA), spike protein or an F protein.

According to an embodiment of the present invention, the virus of the present invention may include a surface protein that is attached to the cell membrane of a host cell, resulting in membrane fusion. Specifically, the virus of the present invention may include a surface protein with a property in which the surface protein present in an inactive form is transformed in an active form by a biomolecule to cause attachment and/or membrane fusion of a host cell to the cell membrane.

According to an embodiment of the present invention, the "virus" may be influenza virus or highly pathogenic influenza virus. The influenza virus includes a glycoprotein, hemagglutinin (HA) as a "surface protein" thereof. Hemagglutinin is expressed as one polypeptide (precursor HA0), and a cleavage site of $HA_0$ is divided into $HA_1$ and $HA_2$ through proteolysis, and in an acidic condition, a fusion peptide composed of hydrophobic amino acid residues is activated. HA has a different amino acid composition from the amino acids of the cleavage site due to pathogenicity of the influenza virus.

According to another embodiment of the present invention, the "virus" may be a coronavirus. The coronavirus includes spike protein as a surface protein, and the spike protein includes $S_1$ and $S_2$ portions. When the $S_1$ portion of the spike protein binds to a host cell, it is cleaved into $S_1$ and $S_2$ by a protease, and a hydrophobic domain at the end of $S_2$ is exposed and thus activated.

According to still another embodiment of the present invention, the "virus" may be a paramyxovirus. The paramyxovirus includes an HN protein as a surface protein. The HN protein is a binding (or attaching) protein that binds or attaches the virus to a host cell, and while it is present as a precursor $HN_0$ in the inactive state of the virus, it is activated by removing an amino acid residue from the C-terminus through hydrolysis. Also, a paramyxovirus includes F protein as a surface protein. While the F protein is present as a precursor $F_0$ in the inactive state of the virus, it is activated as a new form of the N-terminus $F_1$ after cleavage into $F_1$ and $F_2$ by a biomolecule. The F protein includes both a polybasic residue and a monobasic residue at the cleavage site. Preferably, the F protein may be an F protein that has a polybasic residue.

The term "reaction" used herein refers to changes in physical and/or chemical states such as condition, color, shape or chemical bond, when any material is in contact with a different material, or a phenomenon thereof.

In one embodiment, when the surface protein of a virus reacts with a biomolecule, the surface protein of a virus is transformed, resulting in a state change to an activated virus such that a fusion peptide is formed. In another embodiment, the reaction between the activated virus and a probe may be fusion or aggregation.

The fusion may be, according to an embodiment, a phenomenon in which the active virus binds to a part of probe of the present invention. It may be understood that the fusion also includes a phenomenon in which a substance in the probe is released to the outside through fusion. In one example, the active virus may be detected by confirming whether the active virus is fused to the probe using a principle that a fusion peptide present in the surface protein of the active virus induces fusion of an endosome to the cell membrane.

The aggregation refers to a phenomenon in which molecules or particles bind together in a solution. In one embodiment of the present invention, the agglutination refers to a phenomenon in which probes are coagulated by reducing the stability of the probe of the present invention due to a virus which is a detection target, resulting in coagulation and/or precipitation.

The term "biomolecule" used herein refers to a molecule required for the structure, function or signal transduction in a living organism. The biomolecule may include a specific structure and/or region for a function or signal transduction. The specific structure for such a biomolecule is a driving force generating a reaction, through an intermolecular binding reaction. The biomolecule includes an amino acid and a protein, a sugar and a carbohydrate, a fatty acid and a lipid, and a nucleotide and a nucleic acid, and encompasses all products made in a living organism and/or artificially synthesized products. According to an embodiment of the present invention, the "biomolecule" may be an enzyme, which is a protein consisting of approximately 62 to 2500 amino acid residues.

In one embodiment, the enzyme may be a protease. In one example, the first synthesized form of the enzyme is inactivated, but in a protein exhibiting activity due to removing(or cleaving) a specific portion, the protease cleaves the specific portion, resulting in activation of the inactivated protein. The protease may be any enzyme capable of cleaving the specific portion without limitation in its type.

In one embodiment, the protease may be one or more selected from the group consisting of furin, trypsin, serine, endoprotease and carboxypeptidase. The protease encompasses derivatives or modifications thereof. For example, the protease may include recombinant human furin, recombinant mouse furin, trypsin-EDTA, acetylated trypsin, N-tosyl-L-phenylalanine chloromethyl ketone-trypsin (TPCK-trypsin), trypsin-acrylic, furin-like protease, Kex2 protease, transmembrane protease serine, TMPRSS2, TMPRSS4, tryptase clara, plasmin, serine protease, subtilisin-like endoprotease, carboxypeptidase and combinations thereof.

In one embodiment, when the trypsin reacts with the surface protein of influenza virus, hemagglutinin (HA), enzymatic degradation of the trypsin occurs at His57-Asp102-Ser195, and enzymatic degradation of the furin occurs at His194-Asp153-Ser368. Since the trypsin degrades both the hemagglutinin of highly and lowly pathogenic influenza virus, the highly/lowly pathogenic influenza virus may be detected using trypsin. In addition, since furin may degrade only hemagglutinin of a highly pathogenic influenza virus, the highly pathogenic influenza virus may be detected using furin.

In another embodiment, when the protease reacts with the surface protein of the coronavirus, hydrolysis occurs at the boundary between the S1 and S2 regions. Therefore, as the hydrophobic domain of S2 is exposed by cleavage through the hydrolysis, the coronavirus is activated for detection using a probe of the present invention.

In yet another embodiment, a biomolecule reacting with the surface protein of a paramyxovirus may be a protease or carboxylpeptidase, which facilitates cleavage at an arginine residue of the C-terminus (carboxyl side). The protease may recognize and cleave F protein containing an R-X-K/R-R arrangement. As a specific example, the protease may be furin or subtilisin-like endoprotease.

The term "active virus" or "activated virus" used herein refers to a virus in which a surface protein thereof is activated by a biomolecule that specifically reacts with the surface protein of the virus. Here, the activation of the surface protein of a virus means that the surface protein of the virus is changed to be ready for the reaction between a host cell and/or a probe, for example, a specific reaction between a host cell and/or a probe. Here, the term "activation of a virus" refers to a process of converting an inactive virus into an active virus. Since the activation of a virus is necessary for infection by a virus, the viral infection may be determined by detecting the presence of the active virus.

In one embodiment, activation of the surface protein of a virus means that a state in which a fusion protein or fusion peptide that is present on the surface protein of the virus is able to fuse with the surface of the membrane of a host cell or the surface of a probe of the present invention.

In one embodiment, for the influenza virus, activated virus means that the conversion of the surface protein hemagglutinin (HA) to expose the fusion peptide present in the hemagglutinin in an inactivated state through degradation by an enzyme. That is, an enzyme for specifically degrading the cleavage site of HA includes furin and/or trypsin. Since furin may degrade only hemagglutinin of a highly pathogenic influenza virus, when HA of the influenza virus is degraded by furin, the influenza virus exhibits high pathogenicity. Also, since trypsin may degrade both hemagglutinin of the highly and lowly pathogenic influenza virus, when the hemagglutinin is degraded by trypsin, the influenza virus exhibit high or lowly pathogenicity. A restriction site of the lowly pathogenic influenza virus includes -R- in the C-terminus, and a restriction site of the highly pathogenic influenza virus consists of R/K-R-K-K-R.

When HA is activated by an enzyme specifically degrading the restriction site of HA, rearrangement of HA occurs, and thus the fusion peptide in HA is exposed to the outside, thereby resulting in an active virus. Likewise, when the influenza virus activated under an acidic condition encounters a probe according to an embodiment of the present invention, the reaction between the influenza virus and the probe occurs due to the fusion peptide of the influenza virus. By measuring signal varying with the probe reaction, a viral infection may be detected.

In another embodiment, for a coronavirus, the activated virus means that a surface protein, i.e., spike protein (S protein), is changed such that a hydrophobic domain present in the spike protein in an inactivated form is exposed due to a biomolecule. Specifically, when an S1 region of the spike protein binds to a host cell, the spike protein is cleaved into $S_1$ and $S_2$ by a protease, and a hydrophobic domain at the end of S2 is exposed, resulting in activation of the virus. The exposed hydrophobic domain of S2 is attached to a hydrophobic domain in the cell membrane of the host cell, and on the other side, membrane fusion takes place due to a conformational change in which the middle part of the S2 region is folded while binding to the outer membrane of the virus. Therefore, when the probe of the present invention reacts with the activated coronavirus, the probe's stability is changed, various signals are generated, and thus a viral infection may be detected by measuring such a change.

In another embodiment, a virus activated in a paramyxovirus refers to a state in which a residue of the C-terminus is removed from a HN protein by a biomolecule. Specifically, while the HN protein retains an inactivated state ($HN_0$), when approximately 90 residues of the C-terminus are removed, the HN protein is activated to allow the paramyxovirus to be attached to the membrane of a host cell or target. Further, active paramyxovirus means that F protein is cleaved into $F_1$ and $F_2$ by a biomolecule. In the inactive paramyxovirus, F protein binds to a biomolecule in a precursor $F_0$ state, the F protein is cleaved into $F_1$ and $F_2$, thereby becoming an active virus capable of fusing with the cell membrane of a host cell. That is, membrane fusion is initiated with formation of an active surface protein containing SS-bonding chain $F_1$ and $F_2$, and fixation of an *N*-terminus residue of $F_1$ exhibiting broad hydrophobicity to the cell membrane of a host cell or a target. Therefore, the paramyxovirus is activated by reacting a biomolecule of the present invention with the surface protein, and the activated virus reacts with the probe of the present invention again, resulting in a change in the probe. Accordingly, the virus may be detected by measuring the change.

The term "probe" used herein refers to a material that reacts with a virus activated by a biomolecule that specifically reacts with a surface protein of a virus, thereby detecting the presence of the virus or an active virus. The probe encompasses any materials that have considerably higher ability to react with the active virus than a material except the virus or an inactive virus, or specifically react with the active virus, thereby capable of significantly detecting the presence of the active virus, and the type and shape thereof are not specifically limited. In the specification, the probe is used in combination with a "detection factor."

The probe of the present invention may have various structures or shapes according to the type of target virus or the surface protein of a virus.

According to an embodiment of the present invention, the probe may have a membrane structure.

The term "membrane structure" used herein refers to a structure in which the inner side thereof is surrounded by a membrane or shell. The membrane structure includes any type of encapsulation particles such as vesicles, artificial cells or microcapsules without limitation. In the specification, the membrane structure is used in combination with a shell structure. The membrane structure also includes a structure containing a fluid or a separate composition inside.

The membrane includes a mono layer or bilayer structure. The mono layer may be a membrane structure including "hydrophobic domain-hydrophilic domain," and in one embodiment, the membrane structure including a hydrophobic core and a hydrophilic shell in an aqueous solution. The bilayer is a membrane structure including "hydrophilic domain-hydrophobic domain-hydrophobic domain-hydrophilic domain." In one embodiment, the bilayer may include two mono layers which bind together. Also, the bilayer may be a structure including a hydrophilic core, a hydrophobic domain surrounding the hydrophilic core and a hydrophilic shell surrounding the hydrophobic domain.

The membrane structure includes both a single membrane and a multi membrane having two or more single membranes. In one example, when a particle having a single membrane is surrounded by one single membrane again, the particle may have a multi membrane. The single membrane and the multi membrane are concepts distinct from the mono layer and the bilayer. Specifically, a particle including only one membrane with a bilayer structure has a single membrane, and a particle in which a membrane with a mono layer structure is surrounded again by a membrane with a mono layer structure refers to a particle with a multi membrane structure.

A probe with the above-described membrane structure may be prepared by a conventional method included in the present invention, for example, according to an embodiment, microfluidization using micro fluidization equipment, high-pressure homogenization, emulsion or sonication, but the present invention is not limited thereto.

All types of particles with the above-described membrane structure may be included in the probe of the present invention without limitation. In one embodiment, particles may be selected from the group consisting of vesicles, micelles, polymersomes, colloidsomes, liposomes, droplets and combinations thereof, but the present invention is not limited thereto.

The term "micelle" refers to a particle having a hydrophobic core and a hydrophilic shell. In one embodiment, the micelle may be a micelle of a surfactant or a polymer self-assembled micelle. The micelle made by self assembly may have various shapes according to the type of polymer forming a copolymer, but the present invention is not limited thereto. The micelle also includes a multi membrane including two or more mono layers. The shape of the micelle may be, but not limited to, for example, a sphere, ellipsoid or cylinder. A probe with the micelle structure may further include components such as a marker and an inorganic particle in a hydrophobic core region.

The term "polymersome" refers to a particle which has a bilayer membrane structure with "hydrophilic domain-hydrophobic domain-hydrophobic domain-hydrophilic domain," and includes a polymer or copolymer in the hydrophobic and hydrophilic regions. The polymer or copolymer may include an artificially synthesized polymer or copolymer and a lipid, or only an artificially synthesized polymer or copolymer. The artificially synthesized polymer or copolymer refers to any polymer or copolymer, not made of natural lipid. The polymersome comprises a single membrane having a bilayer structure, or a multi membrane having two or more single layer structure.

The term "liposome" refers to a particle having at least one lipid bilayer. The liposome includes both a single membrane and a multi membrane as long as it has a lipid membrane mimicking an amphipathic biomembrane. A method for forming a liposome is well known in the art, and conventionally, a phospholipid may be obtained by suspension in a salted aqueous solution, or sonication with respect to an aqueous solution containing the phospholipid, but the present invention is not limited to the above-mentioned method.

The "colloidsome" refers to a colloidal particle with a size of 1 to 1000 nm or a construct in which the particles are densely packed. The colloidsome may include a mono layer and a bilayer according to types of polymers in hydrophilic and hydrophobic domains, which form a membrane. Also, the colloidsome may include both a single membrane and a multi membrane.

The "droplet" refers to a water drop-like particle in the membrane-structure particles. The droplet includes a mono layer and a bilayer, and both a single membrane and a multi membrane.

A probe of the present invention may be classified according to shape, but the present invention is not limited thereto. In one embodiment, shapes of the probe include a rod, a sphere, a ring, a flat, a cylinder, an ellipsoid, a shape surrounded by a membrane and a combination thereof, but the present invention is not limited thereto.

The rod shape encompasses straight line shapes such as a stick, a bar, or a pole. The sphere encompasses round shapes including a complete sphere and an incomplete sphere. The ring shape generally means a spherical or round shape with a hollow core. The flat shape means a thin and even plate. According to an embodiment, a flat probe may be formed by placing a flat probe on or outside of a base material or carrier through coating.

The probe of the present invention may be classified according to a method for reacting with a component constituting the probe or an active virus.

In one embodiment of the present invention, the probe may be selected from the group consisting of an amphiphilic particle comprising a marker; an organic and inorganic particle; an antibody; an aptamer; and combinations thereof.

According to an embodiment of the present invention, the "amphiphilic particles with a marker" may be an amphiphilic probe having a marker capable of determining a reaction between the probe and an active virus. The term "amphiphilic" may also be amphipathic, which means having both of hydrophilic and hydrophobic properties. The shape of the amphiphilic probe is not specifically limited as long as the probe exhibits amphiphilicity.

The term "marker" used herein refers to a material capable of detecting a reaction between a probe and an active virus. The type of the marker is not particularly limited and varies according to a change in the probe before and after the reaction, and any marker capable of confirming the changed property through a test may be used without limitation. For example, a marker capable of detecting fusion of the probe may be selected from the group consisting of a self-quenched dye, a fluorescent dye, an electrochemiluminescent material and combinations thereof.

In the present invention, the self-quenched dye refers to a material which is quenched when adjacent to another and, when agglomerated dyes are released to be spread, emits fluorescence through dequenching.

In one embodiment, the self-quenched dye may be one or more selected from the group consisting of 3,3-dioctadecyloxacarbocyanine perchlorate (Dio; Dioc), 3,3 - dioctadecyl-5,5 -di(4-sulfophenyl)oxacarbocyanine sodium salt), 4-(4-(dihexadecylamino)styryl)-N-methylpyridinium iodide (DiA; 4-Di-16-ASP), 4-(4-(didecylamino)Styryl)-N-methylpyridinium iodide (4-Di-10-ASP), 1,1 -dioctadecyl-3,3,3,3 - tetramethylindocarbocyanine perchlorate (DiI), 1,1-dioctadecyl-6,6-di(4-sulfophenyl)-3,3,3,3-tetramethylindocarbocyanine, 4,4-diisothiocyanatostilbene-2,2-disulfonic acid disodium salt (DIDS), 1,1-dioctadecyl-3,3,3,3-tetramethylindotricarbocyanine iodide (DiR), 1,1-dioleyl-3,3,3,3-tetramethylindocarbocyanine, fluorescein, BODYPY, tetramethylrhodamine, octadecylodamine B chloride (R18), Alexa, cyanine, and allopicocyanine. The fluorescent dye may have a mean diameter of 200 nm or less, or 50 to 10000 nm.

In one embodiment, the electrochemiluminescent material includes, but is not limited to, tris(2,2'-bipyridyl)ruthenium(II)[Ru(bpy)32+].

In the present invention, the fluorescent dye is a material emitting fluorescence, which absorbs a wavelength included in light, changes color, and reemits. The fluorescent dye is also called a luminescent dye, and any fluorescent dye well known in the art may be used without limitation. In one embodiment, the fluorescent dye may be one or more selected from the group consisting of Texas red, fluorescein, 4-chloro-7-nitrobenzofurazan (NBD-Cl), luminol, fullerene, and a compound with an aromatic group.

In one embodiment, a phosphorescent dye refers to a material exhibiting a photoluminescent effect by re-emitting light with a wavelength changed from the wavelength of a light source absorbed light. In one example, the phosphorescent dye may be, but is not limited to, a sulfide mineral ($X_mZ_n$, X is a metal element, and Z is a non-metal element) or a sulfide of an alkali earth metal.

In the present invention, the amphiphilic particle refers to a particle having a hydrophilic domain and a hydrophobic domain. There is no limit to the amphiphilic particle as long as a particle has a hydrophilic domain and a hydrophobic domain. The amphiphilic particle includes a hydrophilic polymer, a hydrophobic polymer, a lipid polymer and combinations thereof. The polymer may be included in the form of a copolymer.

In one embodiment, the hydrophilic polymer includes one or more selected from the group consisting of polyalkyleneglycol (PAG), polyacrylic acid (PAA), polyacrylonitrile (PAN), polyethyleneoxide (PEO), polyvinylacetate (PVAc), polyvinylalcohol (PVA), polyvinylpyrrolidone, polyacrylamide and hydrophilic polyamino acid, and derivatives thereof. For example, the hydrophilic polymer may be one or more selected from the group consisting of (mono)methoxypolyethylene glycol, (mono)acetoxypolyethylene glycol, polyethylene glycol, a copolymer of polyethylene and propyleneglycol, polyvinylpyrrolidone, poly(glutamine), polyglutamic acid, polythreonine, poly(asparagine), poly(arginine) and poly(serine). Also, the hydrophilic polymer includes derivatives thereof. For example, a derivative of the polyethylene glycol may be methoxypolyethylene glycol (mPEG).

In one embodiment, the hydrophobic polymer may be any material capable of forming the amphiphilic particle with the hydrophilic polymer without limitation. For example, the hydrophobic polymer may be one or more selected from the group consisting of polyester, poly(anhydride), hydrophobic poly(amino acid), polyorthoester and polyphosphazene. The poly(amino acid) includes one or more selected from the group consisting of polyleucine, polyisoleucine, polyvaline, polyphenylalanine, polyproline, polyglycine, polytryptophane, polyalanine, polylactide, polyglycolide, polycaprolactone, and polymethionine. Also, the hydrophobic polymer includes derivatives thereof.

The term "lipid" or "lipid polymer" used herein encompasses phospholipids, lipid proteins, glycolipids, and cationic lipids as long as they are able to form a bilayer membrane structure, but the present invention is not limited thereto. Also, the lipid encompasses a naturally-induced lipid and a synthetic lipid derivative. The phospholipids include glycerophospholipids and phosphosphingolipids. The glycerophospholipids may include a diacrylglyceride structure, and specifically, include posphatidic acid (PA), lecithin (phosphatidylcholine, PC), cephalin and phosphoinositides. The cephalin phospholipids include phosphatidylserine (PS) and phosphatidylethanolamine (PE). Also, the phosphoinositide-like phospholipids include phosphatidylinositol (PI), phosphatidylinositol phosphate (PIP), phosphatidylinositol bisphosphate (PIP2) and phosphatidylinositol triphosphate (PIP3). The sphingophospholipids include ceramide phosphorylcholine (sphingomyelin, SPH), ceramide phosphorylethanolamine (sphingomyelin, Cer-PE) and ceramide phosphoryllipid.

There is no limit to the type of synthetic phospholipid derivative, but in one embodiment, the synthetic phospholipid derivative may be selected from the group consisting of 1,2-didodecanoyl-sn-glycero-3-ethylphosphocholine (EPC), 11,2-dioleoyl-sn-glycero-3-phosphocholine (DOPC), 1-palmitoyl-2-oleoyl-sn-glycero-3-phosphoethanolamine (POPE), 1-palmitoyl-2-oleoyl-sn-glycero-3-phospho-L-serine (POPS) and combinations thereof.

According to an embodiment of the present invention, the amphiphilic particle may be prepared by a known method without limitation. For example, the amphiphilic particle may be prepared by a method for dispersing an amphiphilic block copolymer including a hydrophilic domain and a hydrophobic domain in an aqueous solution and performing sonication, a method for dispersing or dissolving an amphiphilic block copolymer including a hydrophilic domain and a hydrophobic domain in an organic solvent and extracting or evaporating the organic solvent with an excess amount of water, a method for dialyzing an organic solvent with an excess amount of water after dispersion or dissolution of an amphiphilic block copolymer including a hydrophilic domain and a hydrophobic domain in an organic solvent, a method for dispersing or dissolving an amphiphilic block copolymer including a hydrophilic domain and a hydrophobic domain in an organic solvent and vigorously evaporating the solvent using a homogenizer or a high pressure emulsifier, or thin film hydration.

A probe having various structures and shapes may be formed according to the type of polymer used in the amphiphilic particle and a preparation method.

In one embodiment of the present invention, the amphiphilic particle may be a particle having a membrane structure, which is specifically selected from the group consisting of a micelle, a polymersome, a colloidsome, a vesicle, a liposome, a droplet, and combinations thereof.

In one embodiment of the present invention, when the mass fraction calculated according to the following equation is 0.25 to 0.40, the amphiphilic particle may be formed in a polymersome structure, and when the mass fraction is more than 0.40 and 0.70 or less, the amphiphilic particles have a micelle structure.

[Equation 1]

$$\text{Mass Fraction} = \text{Mass of Hydrophilic Polymer}/(\text{Mass of Hydrophilic Polymer} + \text{Mass of Hydrophobic Polymer})$$

Also, when the mass fraction is represented as mass % (percent), the mass% satisfies 25% to 40%, the amphiphilic particles are formed in a polymersome structure, and when the mass% is more than 40% and 70% or less, the amphiphilic particles are formed in a micelle structure. In one embodiment, the size of the amphiphilic particles is not limited. The amphiphilic particles may be nano or micro particles, and for example, the mean diameter of the amphiphilic particles may be 50 to 10000 nm.

According to another embodiment of the present invention, the probe may be "organic/inorganic particles."

In the case of the organic/inorganic particle probe, the stability of the probe in a solution is lowered by the reaction between the probe and an active virus or activated surface protein, resulting in agglomeration of the mixture. Therefore, according to the presence or absence of the reaction, the organic/inorganic particle probe of the present invention may be used to detect a virus.

The term "organic/inorganic particle" used herein refers to a particle including an organic material and an inorganic material. Specifically, such a particle may be a particle in which the surface (or shell) of a core formed of an inorganic material is coated with or bound by an organic material. In one embodiment, the shape of the organic/inorganic particles may be, but not limited to, a rod, sphere, cylinder, flat, ellipsoid or ring.

The inorganic material may consist of a metal, and the metal may be, for example, gold, silver, a mixture including them, and combinations thereof. The metal is preferably gold, silver and a combination thereof.

The organic material may be a stabilizer for an inorganic material. The term "stabilizer" used herein refers to a material for preventing agglomeration of particles and is used in combination with a dispersant. The stabilizer is preferably a stabilizer for a metal. The stabilizer is included regardless of its type, as long as it retains the inorganic material well dispersed in a solvent and may be, for example, a surfactant.

The surfactant is not limited to specific examples and encompasses a cationic surfactant and a non-ionic surfactant. Specifically, the surfactant may be one or more selected from the group consisting of cetyltrimethylammonium bromide, (CTAB, hexadecyltrimethylammonium bromide), tetradecyl trimethyl ammonium bromide (TTAB), dodecyltrimethylammonium bromide (DTAB), polyoxyethylene(20) sorbitan monolaurate (Tween 20), polyoxyethylene(20) sorbitan monopalmitate (Tween 40), (polyoxyethylene(20) sorbitan monooleate (Tween 80) and Triton X-100.

According to an embodiment of the present invention, the organic/ inorganic particle may further include an amphiphilic polymer. The amphiphilic polymer may either bind to the surface of the organic/inorganic particle, or the organic/inorganic particle may be coated with an amphiphilic polymer layer or an amphiphilic polymer shell structure.

According to an embodiment of the present invention, the organic/inorganic particle further including the amphiphilic polymer may further include a functional group layer between the amphiphilic polymer and the surface of the organic/inorganic particle. As a specific example, the functional group layer may allow a compound having a functional group easily binding to the surface of an inorganic particle to bind, and a structure in which a polymer layer is bound to the compound may be formed.

Any compound having the functional group may be used as long as it is able to make an -SH bond to the surface of an inorganic particle and may be, for example, cysteamine, but the present invention is not limited thereto.

In one embodiment of the present invention, when the inorganic particle is gold, a functional group layer may be formed by binding a compound capable of making an SH bond to the surface of the gold particle, and then binding the functional group with an amphiphilic polymer.

According to an embodiment of the present invention, the organic/inorganic particle including an amphiphilic polymer may further include a functional group for initiating amino acid polymerization on the bond between a functional group layer and the amphiphilic polymer. The functional group may be $NH_2$.

The amphiphilic polymer may be formed in an A-B type amphiphilic block copolymer including a hydrophilic A block and a hydrophobic B block or a B-A-B type tri-block copolymer. Also, the amphiphilic polymer may be a lipid.

In one embodiment, the hydrophilic A block may be a hydrophilic polymer, which is one or more selected from the group consisting of polyalkyleneglycol (PAG), polyethylene glycol (PEG), polyvinylalcohol (PVA), polyvinylpyrrolidone, polyacrylamide, polyacrylic acid (PAA), polyacrylonitrile (PAN), polyethyleneoxide (PEO), poly(vinylacetate) (PVAc), hydrophilic poly(amino acid) and a derivative thereof. The hydrophilic A block is preferably one or more selected from the group consisting of monomethoxy polyethylene glycol, monoacetoxy polyethylene glycol, polyethylene glycol, a copolymer of polyethyelne and propyleneglycol, polyglutamine, polyglutamic acid, polythreonine, poly(asparagine), poly(arginine), poly(serine) and polyvinylpyrrolidone. The hydrophilic A block has a number average molecular weight at 200 to 50,000 daltons or 1,000 to 20,000 daltons.

In one embodiment, the hydrophobic B block may be a hydrophobic polymer. Specifically, the hydrophobic B block may be one or more selected from the group consisting of polyester, poly(anhydride), hydrophobic poly(amino acid), polyorthoester and polyphosphazene. The hydrophobic B block is preferably one or more selected from the group consisting of polyleucine, polyisoleucine, polyvaline, polyphenylalanine, polyproline, polyglycine and polymethionine, polytryptophane, polyalanine, polylactide, polyglycolide, polycaprolactone, polydioxane-2-one, a copolymer of polylactide and glycolide, a copolymer of polylactide and dioxane-2-one, a copolymer of polylactide and caprolactone, and a copolymer of polyglycolide and caprolactone. The hydrophobic B block has a number average molecular weight at 50 to 50,000 daltons or 200 to 20,000 daltons.

The organic/inorganic particle may be prepared by reacting an ionic inorganic material with a reducing agent capable of reducing the inorganic material in the presence of the amphiphilic polymer as the stabilizer. As the stabilizer used to prepare the organic/inorganic particle, cetyltrimethylammonium bromide is included, but the present invention is not limited thereto. Also, when the organic/ inorganic particle further includes the amphiphilic polymer layer, the amphiphilic polymer layer preferably has a bilayer structure capable of serving as the stabilizer for the organic/ inorganic particle.

In one embodiment, the organic/inorganic particle may, but not limited to, be nano- or micro-scale. For example, the organic/inorganic particle may have a mean diameter at 50 to 1000 nm.

The organic/inorganic particle of the present invention may be prepared using an emulsion, thin film hydration or dialysis, and specific preparation conditions may vary within a conventionally used range according to types of organic material, inorganic material and polymer.

The organic/inorganic particle of the present invention may further include a marker for more accurately determining the presence or absence of a reaction with a virus.

According to yet another embodiment of the present invention, the probe may be an "aptamer." The term "aptamer" used herein refers to a material capable of binding to a target molecule in the form of single- or double-stranded DNA or RNA.

According to yet another embodiment of the present invention, the probe may be an "antibody." The term "antibody" used herein refers to a material capable of binding to a target molecule and may be a protein binding to an amino acid with a sugar chain.

The target molecule of the present invention may be an active virus, an activated surface protein, a protein exhibiting antigenicity of the active virus and combinations thereof. The aptamer or antibody may specifically bind to the target molecule and preferably have a structure that can complementarily bind to the target molecule.

The aptamer or antibody may detect a virus according to the presence or absence of a specific bond between the aptamer or antibody and an active virus, an activated surface protein and/or an antigenic protein of the active virus.

In one embodiment of the present invention, the target molecule may be a membrane fusion protein present on the surface protein of a virus. Also, in one embodiment, in the case of influenza virus, the target molecule may be the fusion peptide of hemagglutinin (HA). An aptamer or antibody for detecting the fusion peptide of HA includes a complementary structure or sequence capable of specifically binding to the fusion peptide. Also, in the case of coronavirus, the target molecule may be a spike protein, and in the case of paramyxovirus, an F protein.

The aptamer or antibody may further include a metal particle or a marker in order to more easily or clearly confirm the binding reaction with a target molecule.

A method for preparing an aptamer or antibody according to an embodiment of the present invention is well known in the art and may be used to prepare and select an aptamer or antibody by a known method. Also, a method for confirming the binding of an aptamer or antibody is well known in the art. For example, when an aptamer or antibody binds to a gold particle, color is changed according to the change in size of the particle through the binding of the aptamer or antibody binding to the metal particle to a fusion peptide of a virus. An active virus may be detected by observing such color change.

A kit or composition of the present invention may include either single type or two or more types of probes.

In one embodiment of the present invention, the kit or composition may further include an acid material with pH 6 or less. For example, the acid material with pH 6 or less may be an acid solution, and the acid solution with the above range of pH may be included in the kit and may be directly prepared for use. The acid solution with pH 6 or less may be any solution satisfying the pH condition without limitation. As the acid solution, a stock solution may be commercially available or directly prepared for use. For example, a thick acid solution, a stock solution, may be diluted in water to have pH 6 or less. For example, the acid solution may have pH 4 to 6 or pH 5 to 5.7. Since the above-mentioned range is similar to the pH range in a host cell, detection efficiency may be increased.

In one embodiment, the kit and composition of the present invention may further include an adjuvant.

The adjuvant may be used for a biomolecule to help the reaction with a virus or may help the reaction of a target molecule with a probe. As a specific example, the adjuvant may be used to help the reaction of a protease with a surface protein or to help the reaction of a target molecule including the activated surface protein of an active virus and the antigenic protein of the active virus with a probe.

The term "adjuvant" used herein may be any material that is able to increase detection sensitivity and/or specificity for a virus through the above-described reaction. The detection sensitivity and/or specificity of a virus may be increased by increasing the rate of the reaction, decreasing the minimum value at which the reaction takes place, or inhibiting other reactions except the reaction between a surface protein and a biomolecule, but the present invention is not limited to the above-mentioned mechanisms. The adjuvant encompasses all materials that are able to increase detection sensitivity and/or specificity according to types of biomolecule and surface protein reacting therewith without limitation.

A specific example of the adjuvant may be a ketone.

The ketone compound may encompass, for example, phenylethylchloromethylketone, tosyl phenylalanyl chloromethyl ketone (TPCK) and a combinations thereof, but the present invention is not limited thereto.

In one embodiment of the present invention, for the detection of influenza virus, a ketone may be further be included as an adjuvant. In this case, the sensitivity of the kit may be further increased by reducing the activities of other enzymes and increasing the activity of a hemagglutinin (HA) protease, particularly, trypsin.

The present invention relates to a method for detecting a virus.

The detection method of the present invention includes contacting a sample with a biomolecule, and contacting the sample in contact with the biomolecule with a probe. Specifically, the detection method of the present invention includes contacting a sample obtained from a subject with a biomolecule that specifically reacts with a surface protein of a virus; and contacting the sample in contact with the biomolecule with a probe that reacts with the virus activated by the biomolecule.

The detection method of the present invention may detect a virus by confirming the presence or absence of a reaction occurring due to such contacts. As an example, an active virus may be detected by examining whether a probe is fused with an active virus using the mechanism of the cell membrane of an endosome fusing by a fusion peptide present in the surface protein of the active virus. In another example, an active virus may be detected by examining whether a probe is aggregated using the mechanism in which the stability of the probe is reduced by a fusion peptide present in the surface protein of the active virus.

By a method for examining the reaction in the present invention, a signal or change of the probe generated by the reaction may be measured. As an example, the change or signal generated when the active virus reacts with the probe may be a change in one or more selected from the group consisting of fluorescence intensity, luminescence intensity, phosphorescence intensity, absorbance, electrical signal, surface-enhanced Raman spectroscopy (SERS) signal, field-effect transistor (FET), color and the dispersity of probes, but the present invention is not limited thereto.

For example, when a self-quenched dye is used as a marker capable of determining the reaction of a probe, an active virus is fused with the probe, dequenching occurs by releasing a marker such as a self-quenched dye carried in the probe, and thus fluorescence is emitted. Therefore, the infection by a virus may be detected by measuring the change in fluorescence intensity.

Also, when a fluorescent dye is used as a marker for determining the reaction of a probe, the fluorescent dye carried in the probe may be released due to fusion of an active virus with the probe, and fluorescence intensity may be measured to detect the infection by the virus. Other than fluorescence intensity, since luminescence intensity, phosphorescence intensity and absorbance intensity are changed according to wavelength, like the fluorescence intensity, infection by a virus may be detected by measuring the intensities. Particularly, among such fluorescent dyes, luminol or fullerene may be used for visually detecting a change in color without measuring fluorescence intensity by a special apparatus.

In addition to the fluorescent dye, an electrochemiluminescent material such as tris(2,2'-bipyridyl)ruthenium(II)[Ru(bpy)32+]) may be included, and in this case, the change in color may be visually observed.

Also, a virus may be detected by measuring an electrical signal using a nano gap sensor. The electrical signal may be measured as a current change or by using FET. The nano gap sensor refers to a sensor including an electrode having a gap interval of approximately 100 nm or less. For example, when a fluorescent dye is used as a marker capable of determining the reaction of a probe, the fluorescent dye in the probe is released due to fusion of an active virus with the probe and placed in the nano gap of the nano gap sensor. Here, the viral infection may be detected by measuring a current change of a metal particle such as gold, silver, chromium, titanium, platinum, copper, palladium, indium tin oxide (ITO) or aluminum, which is has been placed in the nano gap sensor in advance. The metal particle may have a mean diameter at several nanometers, for example, 2 to 4 nm.

Further, a virus may be detected by measuring an SERS signal. To measure the SERS signal, a base material capable of binding to a sample may be used. The base material may be a nanoparticle, a colloid, or a liquid, but the present invention is not limited thereto. For example, when a fluorescent dye is used as a marker capable of determining fusion of a probe, the fluorescent dye in the probe is released due to the fusion of an active virus with the probe.

Following binding of the release fluorescent dye with the base material, a metal particle is introduced again to the fluorescent dye bound with the base material. Raman spectroscopy may be amplified by introducing the metal particle such as gold or silver. Afterward, the viral infection may be detected by measuring the Raman spectrum of the fluorescent dye using a Raman spectrometer.

As another example, a virus may be detected by confirming aggregation, that is, a change in dispersity of probes due to the aggregation of the probes by an active virus. For example, when an organic/inorganic particle is used as a probe, the particles are aggregated by reducing the stability of the organic/inorganic particle by the active virus.

According to an embodiment of the present invention, the stability of an organic material is reduced by attracting a stabilizer or amphiphilic polymer such as a surfactant present around an organic/inorganic particle by a fusion peptide present in an active virus, resulting in aggregation of the organic/inorganic particles. As it is easy to visually observe such aggregation, the viral infection may be easily detected.

In one embodiment, at least one of the procedures may be performed under the condition of pH at 6 or less. For example, at least one of the procedures may be performed under an acidic condition such as pH 4 to pH 6 or pH 5 to pH 5.5. In one embodiment of the present invention, the detection of influenza virus in an acidic condition may provide a condition very similar to the environment in a host cell, and thus detection accuracy may be increased.

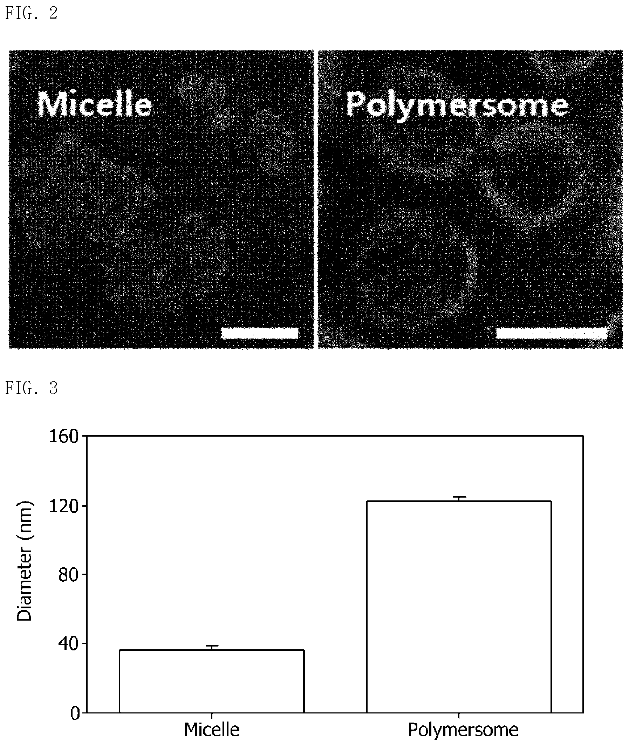

FIG. 3 shows a schematic diagram illustrating a method for detecting influenza virus according to an embodiment of the present invention. Simply, under the condition of pH 6 or less, pH 4 to pH 6, or pH 5 to pH 5.5, a sample obtained from a subject is added to a well in which furin and/or trypsin are present to activate influenza virus present in the sample and then treated with a probe according to an embodiment of the present invention. When influenza virus is present, the reaction between active influenza virus and a probe occurs due to a fusion peptide, and the presence of the influenza virus may be detected by measuring a signal varying according to the reaction. As described above, the reaction between the active influenza virus and the probe may be fusion or aggregation. The reaction between the active influenza virus and the probe and a signal varying thereby are described above.

The term "sample" used herein refers to a material obtained to represent a parent for detection of a virus. In one embodiment, the sample may be saliva, oral mucus or excreta sample.

The present invention also relates to a composition for detecting a virus, which includes a biomolecule that specifically reacts with a surface protein of a virus; and a probe that reacts with the virus activated by the biomolecule. Here, all the details about the kit for detecting a virus and the method for detecting a virus may be applied to the composition for detecting a virus.

Hereinafter, the present invention will be described with reference to examples. The following examples are merely provided to exemplify the present invention, and the scope of the present invention is not limited thereto.

**[Examples]**

**[Preparation Example 1] Preparation of self-quenched dye-carrying probe in the form of host cell-like amphiphilic particle (FluSome)**

To prepare amphiphilic bilayer particles, same as the surface of a host cell, a probe was prepared in the form of a micelle or polymersome-type amphiphilic particle containing a marker using an mPEG-b-PLA copolymer.

Specifically, self-quenched dye-carrying amphiphilic particles were prepared by dispersing mPEG-b-PLA (poly lactic acid) 2 mL, dissolving DiO (Ex: 484nm) and Dil (Em: 565nm) as self-quenched dyes, and dialyzing the resulting solution with excess water. A polymer some was prepared by adjusting the mass fraction between the molecular weights of the mPEG polymer and the polylactide (PLA polymer) to be 0.3, and a micelle was prepared by adjusting the mass fraction to be 0.7, respectively.

Form of the prepared amphiphilic particles were visualized using a transmission electron microscope (TEM).

As a result, as shown in FIG. 2, it could be confirmed that a micelle-type spherical particle having a hydrophobic core and a hydrophilic shell and a polymersome-type spherical particle having a hydrophilic core surrounded by a hydrophobic membrane and a hydrophilic shell were prepared.

Also, the sizes of the prepared amphiphilic particles were measured by dynamic light scattering (DLS).

Consequently, as shown in FIG. 3, the size of the amphiphilic particle was measured at 36.79 ± 1.52 nm for the micelle, and 122.12 ± 2.50 nm for the polymer some.

Also, the molecular weight of the polymer some was 6700 g/mol, and the molecular weight of the prepared micelle was 4500 g/mol.

Also, an amphiphilic particle-type probe was prepared by the same method as described above, except that the copolymer was replaced with an mPEG-b-polyleucine block copolymer.

**[Preparation Example 2] Preparation of organic/inorganic particle probe**

A seed solution was prepared by adding a surfactant hexadecyltrimethylammonium bromide (Sigma Aldrich), as a stabilizer, and a reducing agent sodium borohydride (Sigma Aldrich) to gold chloride trihydrate (Sigma Aldrich) having gold ions, and then a rod-shaped organic/ inorganic particle in which the surface of a gold particle was coated with hexadecyltrimethylammonium bromide as a stabilizer was prepared by sequentially adding the hexadecyltrimethylammonium bromide stabilizer, gold chloride trihydrate (Sigma Aldrich), a reducing agent silver nitrate (Sigma Aldrich), L-ascorbic acid (Sigma Aldrich), and the seed solution.

Through observation, the organic/inorganic particle was formed in a gold nano rod form, and the average size of the particle was 10 nm.

**[Preparation Example 3] Preparation of organic/inorganic particle probe including amphiphilic polymer**

A **organic/inorganic particle** probe including an amphiphilic polymer was prepared by reacting an organic/inorganic particle with an amphiphilic polymer.

Specifically, in the presence of cetyltrimethylammonium bromide (CTAB) as a stabilizer, an organic/inorganic particle was prepared by the same method as described in Preparation Example 2 by reacting gold ions with a reducing agent. Subsequently, an organic/inorganic particle including an amphiphilic polymer was prepared by preparing a copolymer including 4-aminothiophenol and polyphenylalanine in a molar ratio of 1 : 35, mixing and stirring the prepared organic/inorganic particle with an mPEG-pPhe(polyphenylalanine) copolymer to have the polymer adhere to the surface of the gold nano rod. A micelle-type organic/inorganic particle probe in which a gold nano rod-polymer was self-assembled was prepared by dissolving the resulting particle in tetrahydrofuran (THF), evaporating the THF while agitating, forming a thin film along a flask wall, and hydrating the film.

**[Preparation Example 4] Preparation of amphiphilic particle probe in a liposome structure**

A liposome-type probe was prepared by the same method as described in Preparation Example 1, except for the following conditions.

Specifically, a self-quenched dye-carrying liposome particle was prepared by mixing phosphatidyl chloline, cholesterol and monophosphoryl lipid A (MPL) in a weight ratio of 5:2:0.02 to have an organic material at a total of 10 mg and dispersing the resulting mixture in 1 mL of chloroform (CF), evaporating the CF, adding 40 μg each of DiO (Ex: 484nm) and DiI (Em: 565nm) to the formed film with a self-quenched dye (purchased from Avanti), hydrating the resulting product at 60 °C for 12 hours, and stirring the product for 12 hours, and then performing dialysis.

**[Experimental Example 1] Confirmation of fluorescence resonance energy transfer (FRET) effect of FluSome**

To confirm the FRET effect of the amphiphilic particle-type probe (FluSome) prepared by the method described in Preparation Example 1, quenching and dequenching effects were examined in the absence of influenza virus.

In the absence of influenza virus, a dequenching effect was examined using Triton X-100 disrupting particles by degrading a bilayer structure with an anionic surfactant. Fluorescence intensity was measured using a fluorescence measuring device, a hybrid multi-mode microplate reader (Synergy H4, BioTek, USA).

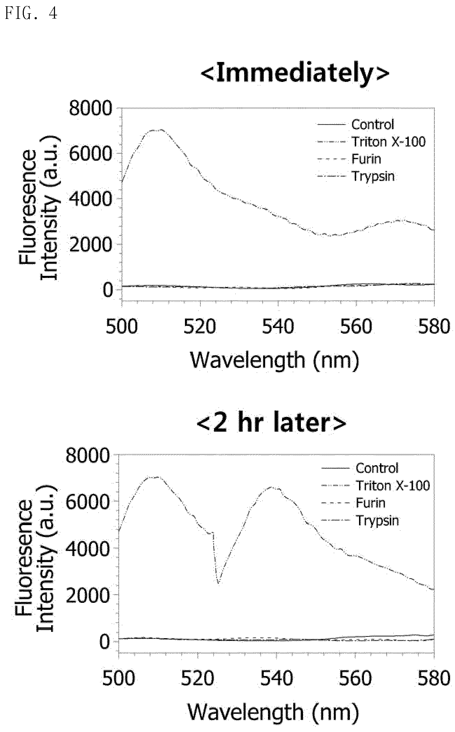

As a result, as shown in FIG. 4, immediately after furin, trypsin or Triton X-100 was treated and 2 hours after the treatment, in the enzyme-free control, the furin-treated group and the trypsin-treated group, there was no change in fluorescence intensity, whereas in the Triton X-100-treated group, the fluorescence intensity was changed. In the furin and trypsin-treated groups, in the absence of influenza virus, virus activation and fusion by a fusion peptide did not occur even with the treatment of furin and trypsin, and therefore there was no change. However, when the amphiphilic particle was destroyed with Triton X-100, there was a change in fluorescence intensity due to dequenching of the self-quenched dye.

**[Experimental Example 2] Detection of influenza virus using FluSome**

An experiment for confirming the ability of the amphiphilic particle-type probe (FluSome) prepared by the method of Preparation Example 1 to detect highly pathogenic influenza virus was conducted.

Specifically, furin capable of specifically degrading hemagglutinin in highly pathogenic influenza virus and trypsin capable of specifically degrading hemagglutinin in lowly and highly pathogenic influenza viruses were used to examine whether highly/lowly pathogenic influenza viruses can be detected according to the treatment with the above-mentioned enzymes.

Lowly pathogenic influenza viruses were provided from Green Cross Veterinary Product Co., Ltd., and highly pathogenic viruses were provided from Hanoi University of agriculture.

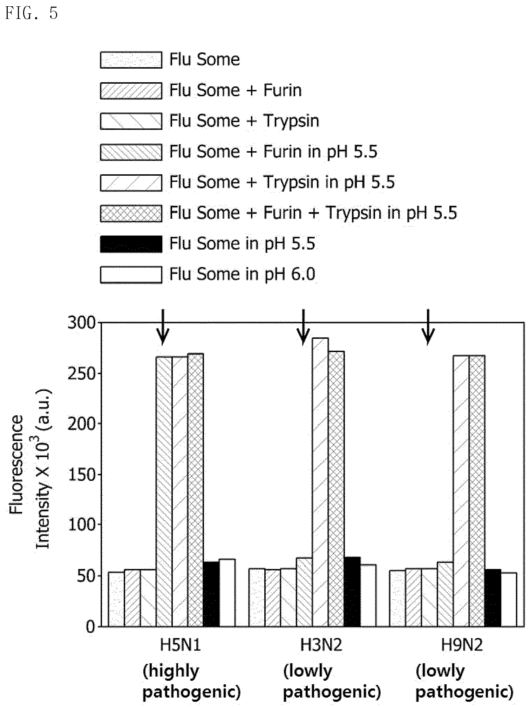

Subjects were divided into a FluSome-only treated group (enzyme-free group) and enzyme-treated groups, and a change in fluorescence intensity of each experimental group in the presence of an acidic condition (pH 5.5) was measured. To reach pH 5.5, an acid stock solution was mixed with a solution containing FluSome. The results of measuring fluorescence intensities are shown in Table 1 and FIG. 5. In Table 1, the first row shows the fluorescence intensity of a highly pathogenic influenza virus, the second row shows the fluorescence intensity of lowly pathogenic influenza virus ($H_3N_2$) and the third row shows the fluorescence intensity of lowly pathogenic influenza virus ($H_9N_2$).

[Table 1]

| FluSome | FluSome +Furin | FluSome + Trypsin | FluSome+ Furin at pH 5.5 | FluSome+ Trypsin at pH 5.5 | FluSome+ Furin+ Trypsin at pH 5.5 | FluSome at pH 5.5 | FluSome at pH 5.0 |
|---|---|---|---|---|---|---|---|
| 54.126 | 55.584 | 56.19 | 266.843 | 266.534 | 268.831 | 62.975 | 65.212 |
| 56.504 | 56.272 | 57.531 | 67.533 | 284.326 | 271.775 | 67.893 | 60.253 |
| 54.389 | 56.563 | 57.382 | 62.971 | 267.604 | 267.84 | 55.447 | 52.827 |

As shown in FIG. 5 and Table 1, in the enzyme-free group and non-acidic group, change in fluorescence of a probe was not observed. However, the highly pathogenic influenza virus groups show, under an acidic condition, that fluorescence is detected by dequenching a self-quenched dye in FluSome when both FluSome and furin were treated, both FluSome and trypsin were treated, and all of FluSome, furin and trypsin were treated under the acidic condition, which indicates that highly pathogenic influenza viruses can be specifically detected using an acidic condition, hemagglutinin protease and FluSome.

**[Experimental Example 3] Detection of various highly pathogenic influenza viruses using FluSome**

To confirm effects of FluSome for detecting various highly pathogenic influenza viruses, the ability of FluSome to detect highly pathogenic influenza viruses was confirmed by the same method as described in Experimental Example 2 with various types of highly pathogenic influenza viruses. All experiments were performed under the same acidic condition (pH 5.5), and total of five types of H5N1- type viruses were used as highly pathogenic influenza viruses.

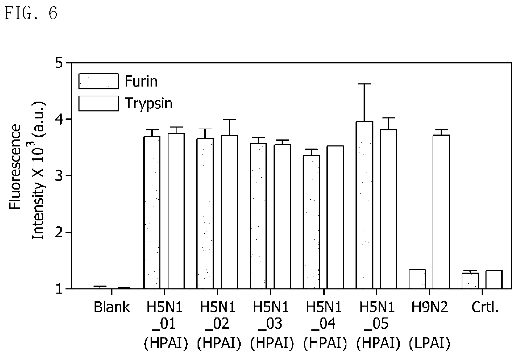

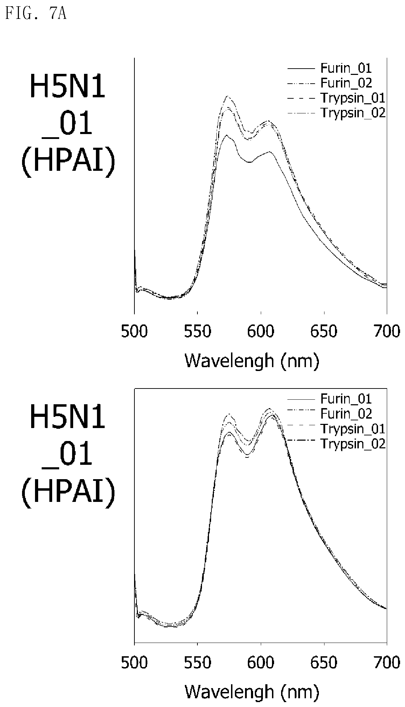

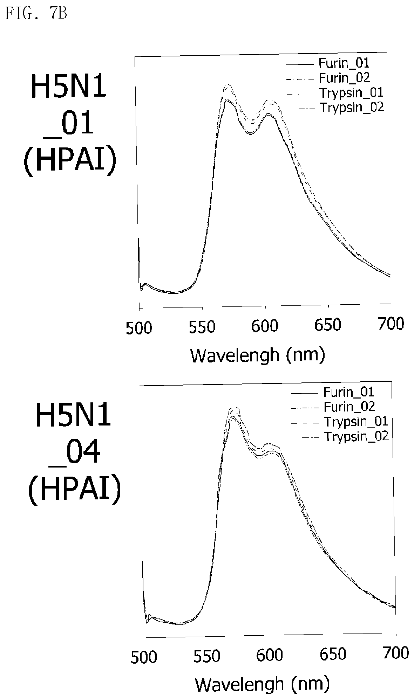

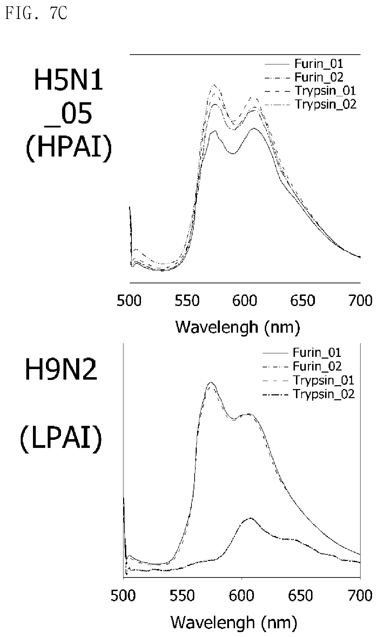

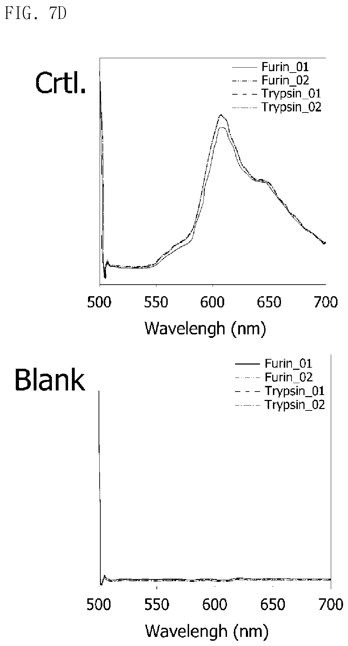

The results of measuring fluorescence intensities are shown in Table 2 and FIGS. 6 and 7.

[Table 2]

|  | Furin | Trypsin |
|---|---|---|
| H5N1_01 | 3346 | 3417 |
| H5N1_02 | 3304 | 3358 |
| H5N1_03 | 3207 | 3185 |
| H5N1_04 | 2948 | 3141 |
| H5N1_05 | 3691 | 3504 |
| H9N2 | 398 | 3376 |
| CONTROL | 318 | 374 |

(continued)

|  | Furin | Trypsin |
|---|---|---|
| BLANK | 21 | 17 |

As shown in Table 2 and FIG. 6, when furin was treated, it was confirmed that fluorescence was detected by dequenching of a self-quenched dye in FluSome.

Also, FIG. 7 shows that when various highly pathogenic influenza viruses were treated with furin or trypsin, fluorescence was detected at approximately 570 nm in all cases, and in the case of lowly pathogenic influenza viruses, fluorescence was detected at approximately 570 nm when treated with only trypsin.

**[Experimental Example 4] Confirmation of detectable concentration of highly pathogenic influenza viruses**

To confirm a detectable concentration of influenza virus using a probe of the present invention, using FluSome according to Preparation Example 1 and highly pathogenic influenza viruses, changes in fluorescence intensity according to the concentration of viruses were measured. As experimental groups, a highly pathogenic influenza virus-treated group and a highly pathogenic influenza+excreta-treated group were prepared, and as controls, an excreta solution-treated group (Excreta Sln), a DPBS-treated group and excreta-treated groups (tested with total of 10 groups according to the types of excreta were prepared. All of the experimental groups and controls were treated with furin under the same pH condition (pH 5.5), and then changes in fluorescence intensity were measured.

The results are shown in Table 3.

[Table 3]

| Virus-treated group (dilution factor) | $1/2^1$ | $1/2^2$ | $1/2^3$ | $1/2^4$ | $1/2^5$ | $1/2^6$ | $1/2^7$ |
|---|---|---|---|---|---|---|---|
| Fluorescence intensity | 118354 | 123582 | 130596 | 150609 | 131845 | 154367 | 111607 |
| Virus-treated group (dilution factor) | $1/2^8$ | $1/2^9$ | $1/2^{10}$ | $1/10^4$ | $1/10^5$ | $1/10^6$ | $1/10^7$ |
| Fluorescence intensity | 11563 | 12011 | 11673 | 11868 | 12109 | 11807 | 11597 |
| Virus-treated group (dilution factor) | $1/10^8$ | $1/10^9$ | $1/10^{10}$ | | | | |
| Fluorescence intensity | 12038 | 10169 | 11439 | | | | |
| Virus+excret a treated group (dilution factor) | $1/10^1$ (virus 50μl+excret a 50μl) | $1/10^2$ (virus 50μl+excret a 50μl) | $1/10^3$ (virus 50μl+excret a 50μl) | $1/10^4$ (virus 50μl+excret a 50μl) | $1/10^5$ (virus 50μl+excret a 50μl) | $1/10^6$ (virus 50μl+excret a 50μl) | $1/10^7$ (virus 50μl+excret a 50μl) |
| Fluorescence intensity | 112252 | 10496 | 10690 | 10183 | 9376 | 9606 | 10637 |
| Virus+excret a treated (dilution factor) | $1/10^8$ (virus 50μl+excret a 50μl) | $1/10^9$ (virus 50μl+excret a 50μl) | $1/10^{10}$ (virus 50μl+excret a 50μl) | | | | |
| Fluorescence intensity | 9901 | 9310 | 10132 | | | | |
| Feces treated group | only excreta_01 | only excreta_02 | only excreta_03 | only excreta_04 | only excreta_05 | only excreta_06 | only excreta_07 |
| Fluorescence intensity | 9111 | 10428 | 10791 | 12149 | 10273 | 11551 | 10651 |
| excreta treated group | only excreta_08 | only excreta_09 | only excreta_10 | excreta sin. | DPBS (control) | | |

(continued)

| | Fluorescence intensity |
|---|---|
| | 10734 |
| | 9901 |
| | 10014 |
| | 11790 |
| | 11412 |
| | |
| | |

As shown in Table 3, it was confirmed that when only influenza viruses were treated in the range of 1/2 to $1/(2^7)$ concentrations (dilution factors), the fluorescence intensities was changed. Further, it was confirmed that, in the influenza virus+excreta-treated group, the fluorescence intensity was changed at 1/10 or higher. This indicates that when influenza viruses were present at 1/2 to $1/(2^7)$ concentrations or a mixture of influenza viruses and excreta was present at 1/10 or higher, it is possible to detect the viruses.

On the other hand, when only excreta was treated, there was no change in fluorescence intensity. This indicates that the change in fluorescence intensity is induced by influenza viruses, and is not influenced by excreta.

**[Experimental Example 5] Detection of influenza virus using organic/inorganic particle including amphiphilic polymer**

To detect the effect of an organic/inorganic particle to detect influenza viruses, efficiency of detecting lowly pathogenic influenza viruses was confirmed using an organic/inorganic particle probe including the amphiphilic polymer prepared by the method described in Preparation Example 3, and enzymes for specifically degrading hemagglutinin of the influenza virus such as furin, trypsin and N-tosyl-L-phenylalanine chloromethyl ketone (TPCK)-trypsin was confirmed.

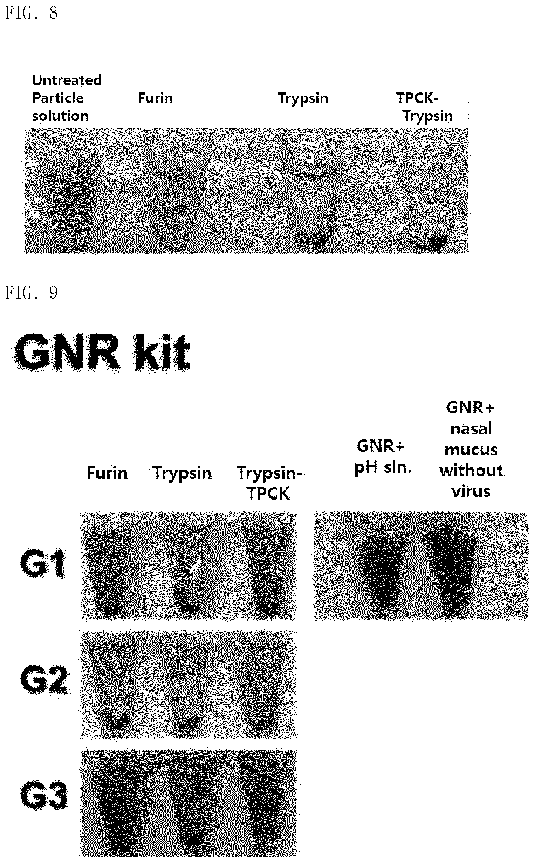

As controls, an enzyme-free group (untreated organic/inorganic particle solution containing amphiphilic polymer) was prepared, and a furin-treated group, a trypsin-treated group and a TPCK-trypsin-treated group were prepared as experimental groups. All of the experimental conditions were performed at pH 5.5, and the control and each experimental group were treated with lowly pathogenic viruses, and then visually observed to examine the changes before and after viral treatment. The results are shown in FIG. 8.

As shown in FIG. 8, in the enzyme-tree control, no apparent change was observed, but in the TPCK-trypsin-treated group, precipitation and a cluster created by decreasing the stability of the organic/inorganic particle including an amphiphilic polymer by viruses are clearly confirmed through visual observation.

**[Experimental Example 6] Detection of influenza viruses using organic/inorganic particle**

Since aggregation of organic and inorganic particles occurs due to the reaction of a peptide of a virus activated by a hemagglutinin protease with a stabilizer on which an organic/inorganic particle probe (GNR) is present, in the presence of a virus, the virus may be rapidly detected according to aggregation. Therefore, an experiment was performed to confirm efficiency of such an organic/inorganic particle for detecting influenza virus.

A rapid kit was used to confirm the efficiency of detecting highly pathogenic influenza viruses (H5N1, H5N6) with the organic/inorganic particle probe (GNR) prepared by the method of Preparation Example 2, and furin, trypsin and TPCK-trypsin, which are enzymes for specifically degrading the hemagglutinin of influenza virus. G1 and G2 indicated the detection of highly pathogenic influenza viruses, and G3 indicated the detection of lowly pathogenic influenza viruses.

Also, GNR was added to a negative control under an enzyme-free condition while an enzyme and a pH solution were added to a nasal mucus taken from the nose of a beagle to check whether the reaction occurs. The result is shown in FIG. 9.

As shown in FIG. 9, while, in the probe-free negative control and enzyme-free negative control, color change was not observed, in both the G1 and G2 groups, color changes were visually observed, and in the lowly pathogenic G3 groups, detectable levels of changes were visually detected, although a color change was detected at a low level when only furin was included. Therefore, it was confirmed that when the organic/inorganic particle probe of the present invention was used, the presence or absence of influenza viruses was rapidly detected.

**[Experimental Example 7] Confirmation of ability to detect influenza viruses according to treatment of enzyme adjuvant**

An experiment was performed to confirm a change in detection efficiency when an adjuvant of the enzyme specifically degrading the hemagglutinin of a virus was further included.

As an enzyme adjuvant, a ketone was used, and the probe (FluSome) prepared according to the method of Preparation Example 1 was used. The experiment was performed by the same method as described in Experimental Example 2, except the ketone was further included. A group treated with the enzyme adjuvant, ketone was represented as Try-TPCK.

[Table 4]

| | | $2^0 =1$ | $2^{-1} =1/2$ | $2^{-2} =1/4$ | $2^{-3} =1/8$ | $2^{-4} =1/16$ | $2^{-5} =1/32$ | $2^{-6} =1/64$ | $2^{-7} =1/128$ | $2^{-8} =1/256$ | $2^{-9} =1/512$ |
|---|---|---|---|---|---|---|---|---|---|---|---|
| **G1 (H5N1_01)** | **Furin** | **88103** | **87389** | **91459** | **91199** | **2863** | **3083** | **5692** | **2353** | **3800** | **4368** |
| | Trypsin | 85807 | 99558 | 92991 | 92397 | 3195 | 2204 | 2338 | 5730 | 1916 | 3338 |
| | Try-TPCK | 91665 | 91665 | 91665 | 93022 | 92688 | 1264 | 1461 | 1587 | 2424 | 3385 |
| G2 (H5N1_02) | Furin | 90707 | 90707 | 96631 | 90745 | 92015 | 1257 | 3699 | 1715 | 3169 | 1739 |
| | Trypsin | 95223 | 95427 | 97742 | 97646 | 93160 | 4369 | 2889 | 1497 | 2920 | 3073 |
| | Try-TPCK | 93914 | 94982 | 96922 | 99526 | 91492 | 2334 | 3837 | 1433 | 2096 | 2807 |
| G3 (H5N6) | Furin | 95180 | 98110 | 99975 | 93760 | 92978 | 6539 | 2526 | 5138 | 2533 | 5519 |
| | Trypsin | 93049 | 94767 | 94093 | 94367 | 4976 | 4808 | 4921 | 5566 | 4803 | 1985 |
| | Try-TPCK | 97286 | 98414 | 97552 | 94610 | 90354 | 93251 | 4996 | 2567 | 4725 | 4885 |

[Table 5]

| Without enzyme | |
|---|---|
| G1 | 6623 |
| G2 | 6868 |
| G3 | 6724 |

[Table 6]

| Without Virus (nasal mucus) | |
|---|---|
| Furin | 6226 |
| Trypsin | 6251 |
| Try-TPCK | 6238 |

As shown in Tables 4 to 6, it was confirmed that all experimental groups treated with Try-TPCK exhibited excellent detection efficiency, compared with a group treated only with furin or trypsin. It is determined that this is because the enzyme adjuvant reduces the activities of other enzymes, except the hemagglutinin protease, thereby increasing the activity of the hemagglutinin protease, and thus the use of such enzyme adjuvant can further enhance the virus detection efficiency, thereby enabling detecting a small amount of viruses and increasing the sensitivity of the detection kit.

**[Experimental Example 8] Detection of influenza viruses through measurement of surface-enhanced Raman scattering signal**

To confirm the detection of influenza viruses according to a measurement of a surface-enhanced Raman scattering signal, instead of the self-quenched dye prepared in Preparation Example 1, FluSome prepared to carry a Raman reporter, 2-naphthalenethiol reacted with a virus sample, and released 2-naphthalenethio was conjugated with nano-scale gold, followed by an observation of a change of a Raman scattering signal under the following measurement conditions: Laser: 785 nm, Power: 0.7 mW, Grating: 300 gr/mm, Hole: 500 um, Spectral range: 300 to 1700 cm-1, Exposure time: 120 sec

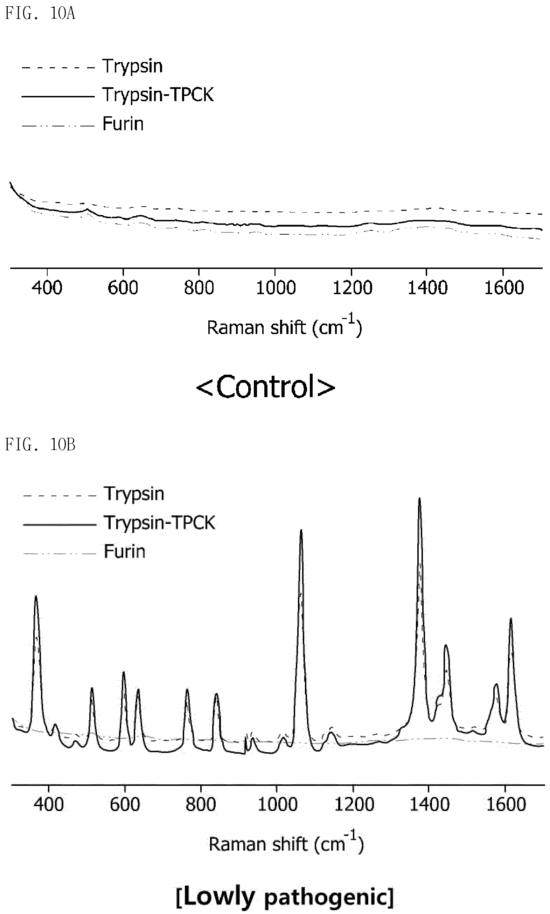

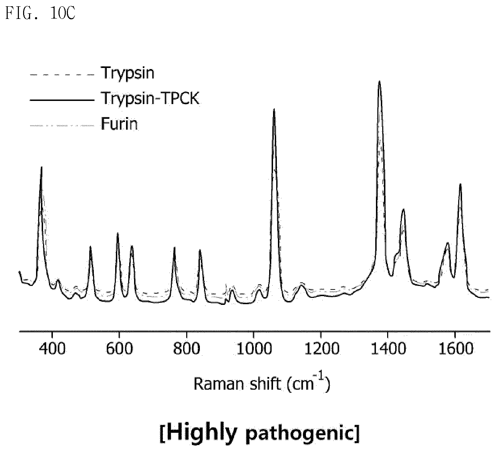

As a result, as shown in FIG. 10, compared with a virus-free control, in trypsin and trypsin-TPCK-treated lowly and highly pathogenic virus groups, according to the existence of a 2-naphthalenethiol-gold conjugate, a change of a Raman signal was confirmed. For furin, only in a highly pathogenic virus group, a change of a Raman signal was confirmed.

Such results show that, through the measurement of surface-enhanced Raman scattering signals, the presence of highly pathogenic influenza viruses can be detected with furin, and the presence of lowly/highly pathogenic influenza viruses can be detected with trypsin.

**[Experimental Example 9] Detection of influenza viruses according to visual observation of color change**

To confirm whether influenza viruses can be detected by visual observation of a color change, a virus sample reacted with FluSome prepared to carry fullerene (TCI) instead of the self-quenched dye prepared in Preparation Example 1, and the color change was observed after the released fullerene was added to a toluene solution. Fullerene refers to a molecule in which carbon elements are arranged in a spherical, ellipsoidal or cylindrical shape.

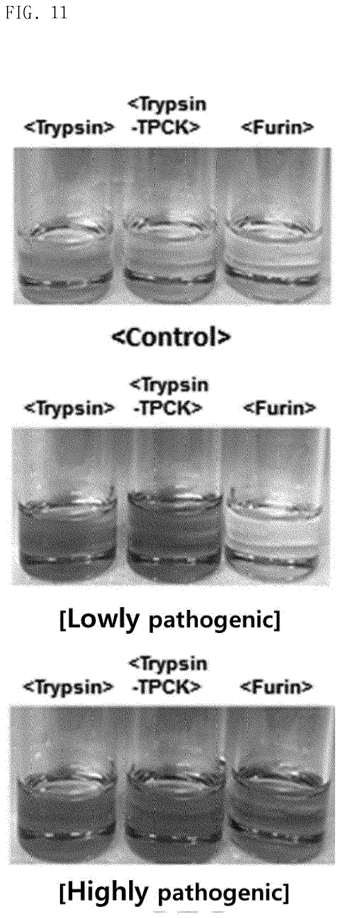

The observation shows that, as shown in FIG. 11, compared with the virus-free control, in the trypsin and trypsin-TPCK-treated lowly and highly pathogenic virus groups, due to the presence of the fullerene, a color change was visually confirmed. For furin, a color change was confirmed only in a highly pathogenic virus group.

Such results indicate that, through visual observation of the color change using fullerene, furin can detect the presence of highly pathogenic influenza viruses, and trypsin can detect the presence of lowly/highly pathogenic influenza viruses.

**[Experimental Example 10] Detection of influenza virus according to measurement of fluorescence change**

To confirm whether influenza viruses can be detected by measuring a fluorescence change using a fluorescent dye, a virus sample reacted with FluSome prepared to carry a fluorescent dye, luminol (Sigma), instead of the self-quenched dye prepared in Preparation Example 1, and following the peroxidation of the released luminol in an alkali aqueous solution, the fluorescence change (Ex: 303 nm, Em: 425 nm) was measured using a hybrid multi-mode microplate reader (Synergy H4, BioTek, USA). Since luminol emits purplish blue fluorescence due to the peroxidation in an alkali aqueous solution, the presence of viruses may be detected by the measurement of a fluorescence change.

Consequently, as shown in Table 7, compared to a virus-free control, in trypsin and trypsin-TPCK-treated lowly and highly pathogenic virus groups, a change in fluorescence intensity according to the peroxidation of luminol was confirmed. For furin, a change in fluorescence intensity was confirmed only in a highly pathogenic virus group.

[Table 7]

|  | Control | Low-pathogenic | High-pathogenic |
|---|---|---|---|
| Trypsin | 3083 | 88115 | 78195 |
| Trypsin-TPCK | 2204 | 97654 | 94257 |
| Furin | 1916 | 2438 | 81248 |

Such results indicate that through the measurement of the fluorescence intensity using luminol, furin can detect the presence of highly pathogenic influenza viruses, and trypsin can detect the presence of mildly/highly pathogenic influenza viruses.

**[Experimental Example 11] Detection of influenza viruses using liposome**

To confirm whether influenza viruses can be detected using liposomes as amphiphilic particles, a virus sample was reacted with the liposome prepared in Preparation Example 4, and change in fluorescence intensity was measured by the same method described in Experimental Example 3.

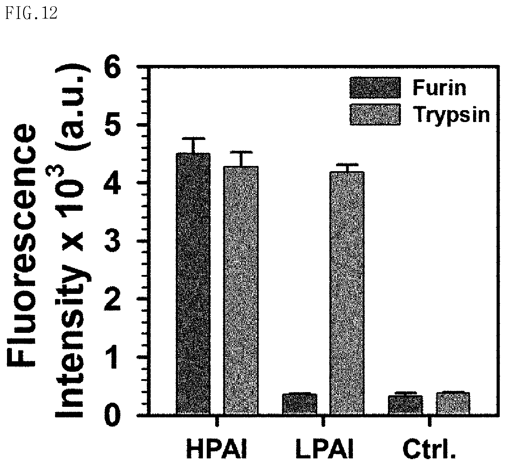

The measurement shows that, as shown in FIG. 12, compared to a virus-free control, in lowly and highly pathogenic virus groups, fluorescence intensities are changed with the treatment of trypsin. For furin, the change in fluorescence intensity was confirmed only in a highly pathogenic virus group.

Such results indicate that, using the liposome as an amphiphilic particle, furin can detect the presence of highly pathogenic influenza viruses, and trypsin can detect the presence of lowly/highly pathogenic influenza viruses.

**Claims**

1. A kit for detecting a virus, comprising:

    a biomolecule that specifically reacts with the surface protein of a virus; and
    a probe that reacts with the virus activated by the biomolecule.

2. The kit of claim 1, wherein the virus is influenza virus, coronavirus or paramyxovirus.

3. The kit of claim 1 or 2, wherein the surface protein of a virus is hemagglutinin (HA), spike protein or F protein.

4. The kit of claim 1 or 2, wherein the biomolecule that specifically reacts with the surface protein of a virus is an enzyme.

5. The kit of claim 4, wherein the enzyme includes one or more selected from the group consisting of furin, trypsin, serine, endoprotease and carboxypeptidase.

6. The kit of claim 5, wherein the enzyme includes one or more selected from the group consisting of recombinant human furin, recombinant mouse furin, trypsin-EDTA, acetylated trypsin, TPCK-trypsin, trypsin-acrylic furin-like protease, Kex2 protease, transmembrane protease serine, TMPRSS2, TMPRSS4, tryptase clara, plasmin, serine protease, subtilisin-like endoprotease and carboxypeptidase.

7. The kit of claim 1, wherein the probe is a micelle, a polymersome, a colloidsome, a vesicle, a liposome or a droplet.

8. The kit of claim 1, wherein the probe is selected from the group consisting of an amphiphilic particle comprising a marker; organic and inorganic particles; an antibody; an aptamer; and combinations thereof.

9. The kit of claim 8, wherein the marker is selected from the group consisting of a self-quenched dye, a fluorescent dye, an electrochemiluminescent material or combinations thereof.

10. The kit of claim 8, wherein the amphiphilic particle includes a hydrophilic polymer and a hydrophobic polymer.

11. The kit of claim 10, wherein the hydrophilic polymer includes one or more selected from the group consisting of polyalkyleneglycol (PAG), polyacrylic acid (PAA), polyacrylonitrile (PAN), polyethyleneoxide (PEO), polyvinylacetate (PVAc), polyvinylalcohol (PVA), polyvinylpyrrolidone and polyacrylamide.

12. The kit of claim 10, wherein the hydrophobic polymer includes one or more selected from the group consisting of polyester, poly(anhydride), polyorthoester, polyphosphazene, polyleucine, polyisoleucine, polyvaline, polyphenylalanine, polyproline, polyglycine and polymethionine.

13. The kit of claim 8, wherein, for the organic and inorganic particles, the inorganic material is one or more selected from the group consisting of gold and silver.

14. The kit of claim 8, wherein, for the organic and inorganic particles, the organic material is a stabilizer of the inorganic material.

15. The kit of claim 14, wherein the stabilizer of the inorganic material is a surfactant.

16. The kit of claim 8, wherein the organic and inorganic particles further include an amphiphilic polymer.

17. The kit of claim 16, wherein the amphiphilic polymer is an A-B type amphiphilic block copolymer including a hydrophilic A block and a hydrophobic B block, a B-A-B type tri-block copolymer or a lipid.

18. The kit of claim 1, further comprising:

    an acid material with pH 6 or less.

19. The kit of claim 1, further comprising:

    an adjuvant for the biomolecule.

20. The kit of claim 19, wherein the adjuvant for the biomolecule is a ketone.

21. A composition for detecting a virus, comprising:

    a biomolecule that specifically reacts with the surface protein of a virus; and
    a probe that reacts with the virus activated by the biomolecule.

22. A method for detecting a virus, comprising:

    contacting a sample obtained from a subject with a biomolecule that specifically reacts with the surface protein of a virus; and
    contacting the sample in contact with the biomolecule with a probe that reacts with the virus activated by the biomolecule.

23. The method of claim 22, wherein the contacts are made under the condition of pH 6 or less.

24. The method of claim 22, further comprising:

    measuring a change in fluorescence intensity of the probe in contact with the sample.

25. The method of claim 22, further comprising:

    measuring a change in surface-enhanced Raman spectrum of the probe in contact with the sample.

**26.** The method of claim 22, further comprising:

measuring a change in the probe in contact with the sample.

**27.** The method of claim 22, further comprising:

observing a color change of the probe in contact with the sample.

**28.** The method of claim 22, further comprising:

confirming whether the probe in contact with the sample is aggregated.

FIG. 1

FIG. 2

FIG. 3

FIG. 4

## \<Immediately\>

## \<2 hr later\>

FIG. 5

FIG. 6

FIG. 7A

FIG. 7B

H5N1
_01
(HPAI)

H5N1
_04
(HPAI)

FIG. 7C

H5N1
_05
(HPAI)

H9N2

(LPAI)

FIG. 7D

EP 3 244 210 A1

FIG. 8

FIG. 9

32

FIG. 10A

Trypsin
Trypsin-TPCK
Furin

Raman shift (cm$^{-1}$)

## \<Control\>

FIG. 10B

Trypsin
Trypsin-TPCK
Furin

Raman shift (cm$^{-1}$)

## [Lowly pathogenic]

FIG. 10C

Legend:
- - - - - Trypsin
——— Trypsin-TPCK
···· Furin

Raman shift (cm$^{-1}$)

**[Highly pathogenic]**

FIG. 11

<Trypsin> <Trypsin -TPCK> <Furin>

<Control>

<Trypsin> <Trypsin -TPCK> <Furin>

[Lowly pathogenic]

<Trypsin> <Trypsin -TPCK> <Furin>

[Highly pathogenic]

FIG.12

## INTERNATIONAL SEARCH REPORT

| | |
|---|---|
| International application No. | |
| **PCT/KR2016/000153** | |

**A.    CLASSIFICATION OF SUBJECT MATTER**

*G01N 33/569(2006.01)i, G01N 33/58(2006.01)i, G01N 21/17(2006.01)i, G01N 21/62(2006.01)i, C12Q 1/37(2006.01)i*

According to International Patent Classification (IPC) or to both national classification and IPC

**B.    FIELDS SEARCHED**

Minimum documentation searched (classification system followed by classification symbols)
G01N 33/569; G01N 33/53; G01N 33/58; G01N 21/17; G01N 21/62; C12Q 1/37

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: virus, detection, diagnosis, influenza, hemagglutinin, enzyme, trypsin

**C.    DOCUMENTS CONSIDERED TO BE RELEVANT**

| Category* | Citation of document, with indication, where appropriate, of the relevant passages | Relevant to claim No. |
|---|---|---|
| X | MITTAL, A. et al., "Kinetics of Influenza Hemagglutinin-mediated Membrane Fusion as a Function of Technique", Analytical Biochemistry, 2002, vol. 303, pages 145-152 See abstract; and pages 145-146. | 1-28 |
| A | GAMBOTTO, A. et al., "Human Infection with Highly Pathogenic H5N1 Influenza Virus", The Lancet, 2008, vol. 371, pages 1464-1475 See the entire document. | 1-28 |
| A | BRANDENBURG, B. et al., "Mechanisms of Hemagglutinin targeted Influenza Virus Neutralization", PloS One, 2013, vol. 8, no. 12, paper number e80034, inner pages 1-14 See the entire document. | 1-28 |
| A | KR 10-2009-0044763 A (REPUBLIC OF KOREA(MANAGEMENT: ANIMAL AND PLANT QUARANTINE AGENCY) et al.) 07 May 2009 See the entire document. | 1-28 |
| A | KR 10-1997-7003432 A (THE MOUNT SINAI SCHOOL OF MEDICINE OF THE CITY UNIVERSITY OF NEW YORK) 03 July 1997 See the entire document. | 1-28 |

| ☐    Further documents are listed in the continuation of Box C. | ☒    See patent family annex. |
|---|---|

| *    Special categories of cited documents: | "T"    later document published after the international filing date or priority date and not in conflict with the application but cited to understand the principle or theory underlying the invention |
|---|---|
| "A"    document defining the general state of the art which is not considered to be of particular relevance | |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
|---|---|
| 19 APRIL 2016 (19.04.2016) | **20 APRIL 2016 (20.04.2016)** |

| Name and mailing address of the ISA/KR | Authorized officer |
|---|---|
| Korean Intellectual Property Office Government Complex-Daejeon, 189 Seonsa-ro, Daejeon 302-701, Republic of Korea | |
| Facsimile No.  82-42-472-7140 | Telephone No. |

Form PCT/ISA/210 (second sheet) (January 2015)

INTERNATIONAL SEARCH REPORT
Information on patent family members

International application No.

**PCT/KR2016/000153**

| Patent document cited in search report | Publication date | Patent family member | Publication date |
|---|---|---|---|
| KR 10-2009-0044763 A | 07/05/2009 | KR 10-0954992 B1 | 29/04/2010 |
| KR 10-1997-7003432 A | 03/07/1997 | EP 0760013 A1 | 09/08/2000 |
| | | JP 10-500574 A | 20/01/1998 |
| | | JP 3621995 B2 | 23/02/2005 |
| | | US 6150131 A | 21/11/2000 |
| | | WO 95-32309 A1 | 30/11/1995 |

Form PCT/ISA/210 (patent family annex) (January 2015)